# EUROPÄISCHE PATENTANMELDUNG

(11) **EP 4 101 423 A1**
(43) Veröffentlichungstag der Anmeldung: **14.12.2022**
(21) Anmeldenummer: 21178977.1
(22) Anmeldetag: 11.06.2021
(51) Int. Cl.: A61F 5/00

(54) **VORRICHTUNG ZUR BEHANDLUNG EINES ORGANISMUS GEGEN GEWICHTSSTÖRUNGEN**

(71) Anmelder: Öztürk, Mustafa, 50733 Köln (DE)
(72) Erfinder: Öztürk, Mustafa, 50733 Köln (DE)
(74) Vertreter: Isarpatent

(57) **Zusammenfassung**

Vorrichtung (1) zur Behandlung eines Organismus gegen Überoder Untergewicht mit einer an einem Körperteil des Organismus tragbaren Einheit, die mindestens eine Aufnahmekammer (3) zur Aufnahme eines Geruchsstoffträgers (4) aufweist, der einen Geruchsstoff zur Beeinflussung einer Nahrungsaufnahme des Organismus freisetzt.

## Beschreibung

Die Erfindung betrifft eine Vorrichtung zur Behandlung eines Organismus gegen Gewichtsstörungen, insbesondere gegen Über- oder Untergewicht.

Übergewicht oder Untergewicht sind bei Menschen und Tieren, insbesondere auch bei Haustieren, weit verbreitet. Ein Großteil der Erwachsenen in Deutschland ist übergewichtig bzw. adipös. Übergewicht bezeichnet einen chronischen Zustand des Körpers mit zu viel Körperfett. Adipositas bzw. Fettleibigkeit bezeichnet ein starkes Übergewicht mit einem Body-Mass-Index BMI von 30 oder mehr. Fettleibigkeit kann zu Folgeerkrankungen führen, beispielsweise Diabetes, Herzkrankheiten oder Schlaganfall. Eine ebenfalls weit verbreitete Ernährungsstörung ist Magersucht bzw. anhaltendes Untergewicht. Bei dieser Störung schränken die betroffenen Personen ihre Nahrungsaufnahme zunehmend ein, da die Betroffenen Angst davor haben, zuzunehmen bzw. zu dick zu sein. Sowohl Fettleibigkeit als auch Magersucht können schwerwiegende Konsequenzen für die Gesundheit der betroffenen Personen haben. Auch Haustiere, insbesondere Katzen oder Hunde, weisen aufgrund einer fehlerhaften Ernährung in vielen Fällen Übergewicht auf. Demgegenüber ist es bei Nutztieren in vielen Fällen gewünscht, das Körpergewicht zu erhöhen.

Es gibt verschiedene konventionelle Ansätze Übergewicht bei Menschen zu behandeln. Beispielsweise beschreibt die EP 2 305 346 B1 eine Vorrichtung zur Behandlung von Übergewicht durch Aktivierung der Schilddrüse. In der DE 19819257 C2 wird eine Vorrichtung beschrieben, bei welcher Übergewicht mithilfe eines Körperübungsgerätes behandelt wird, wobei biometrische Daten des Körpers der betroffenen Person herangezogen werden. Ein weiterer Ansatz wird in der DE 10 2012 015 839 A1 verfolgt. Dabei wird das Übergewicht des Menschen durch Verkleinerung einer Kaufläche behandelt.

Diese herkömmlichen Ansätze weisen unterschiedliche Nachteile auf. In den meisten Fällen kann eine Behandlung nur temporär bzw. über einen kurzen Zeitraum hinweg mithilfe spezieller Geräte ausgeführt werden. Weiterhin verlangen derartige Vorrichtungen die aktive Mitwirkung der betroffenen Patienten, beispielsweise die Durchführung von auferlegten körperlichen-Aktivitäten. Weitere Vorrichtungen, wie beispielsweise Vorrichtungen zur Verkleinerung der Kaufläche, sind unbequem und werden von den Patienten nicht gerne getragen.

Es ist daher eine Aufgabe der vorliegenden Erfindung, eine Vorrichtung zur effizienten Behandlung von Gewichtsstörungen zu schaffen, die die oben genannten Nachteile vermeidet.

Diese Aufgabe wird erfindungsgemäß durch eine Vorrichtung zur Behandlung eines Organismus gegen Über- oder Untergewicht den in Patentanspruch 1 angegebenen Merkmalen gelöst.

Die Erfindung schafft demnach eine Vorrichtung zur Behandlung eines Organismus gegen Über- oder Untergewicht mit einer an einem Körperteil des Organismus tragbaren Einheit, die mindestens eine Aufnahmekammer zur Aufnahme eines Geruchsstoffträgers aufweist, der einen Geruchsstoff zur Beeinflussung einer Nahrungsaufnahme des Organismus freisetzt.

Ein Vorteil der erfindungsgemäßen Vorrichtung besteht darin, dass der Geruchsstoff durch den Geruchsstoffträger kontinuierlich über einen längeren Zeitraum freigesetzt wird und somit das Über- oder Untergewicht langfristig und intensiv behandelt.

Ein weiterer Vorteil der erfindungsgemäßen Vorrichtung besteht darin, dass die Behandlung des Über- oder Untergewichts ohne aktives Zutun des betroffenen Organismus erfolgen kann.

Ein weiterer Vorteil der erfindungsgemäßen Vorrichtung zur Behandlung von Über- oder Untergewicht besteht darin, dass der damit verbundene technische Aufwand gering ist.

Bei einer möglichen Ausführungsform weist die Aufnahmekammer der tragbaren Einheit mindestens eine Membran auf, durch die der von dem Geruchsstoffträger freigesetzte Geruchsstoff an die Umgebungsluft abgegeben wird und zu einem Geruchsorgan des Organismus gelangt.

Bei einer Ausführungsform der erfindungsgemäßen Vorrichtung weist die Aufnahmekammer einen Klapp- oder Drehmechanismus zum Einbringen, Entfernen oder Austauschen eines mit dem Geruchsstoff versetzten Geruchsstoffträgers auf.

Bei einer weiteren möglichen Ausführungsform der erfindungsgemäßen Vorrichtung ist die tragbare Einheit mit der Aufnahmekammer und dem Geruchsstoffträger an einem Kopf des Organismus anbringbar.

Dies bietet zum einen den Vorteil, dass der von dem Geruchsstoffträger freigesetzte Geruchsstoff nur eine geringe Entfernung bis hin zu dem Geruchsorgan des betroffenen Organismus zurücklegen muss. Entsprechend ist die Konzentration des freigesetzten Geruchsstoffes in der Nähe des Geruchsorganes des betroffenen Organismus relativ hoch.

In einer weiteren möglichen Ausführungsform der erfindungsgemäßen Vorrichtung ist die tragbare Einheit mit der darin enthaltenen Aufnahmekammer für den Geruchsstoffträger an einer Ohrmuschel oder an einem Ohrläppchen eines Ohres des Organismus anbringbar.

Das Anbringen der tragbaren Einheit an einem Ohr des Organismus hat einerseits den Vorteil, dass unabhängig von einer Bewegung, beispielsweise einer Kopfbewegung oder einer sportlichen Aktivität der Person, die Aufnahme des freigesetzten Geruchsstoffs durch das Geruchsorgan der Person und der räumliche Abstand der an dem Ohr angebrachten tragbaren Einheit zu dem Geruchsorgan nur geringfügig beeinflusst wird. Darüber hinaus kann die Applikation des freigesetzten Geruchsstoffes kontinuierlich über das Geruchsorgan bzw. die Nase des Organismus gewährleistet werden.

In einer möglichen Ausführungsform wird die tragbare Einheit mithilfe eines Bügels an einer Ohrmuschel des Ohres angebracht. Diese Ausführungsform bietet den besonderen Vorteil, dass die tragbare Einheit der Vorrichtung von vorne nicht sichtbar ist und somit das Erscheinungsbild der betroffenen Person nicht beeinträchtigt.

In einer weiteren möglichen Ausführungsform der erfindungsgemäßen Vorrichtung ist die tragbare Einheit mit der Aufnahmekammer für den Geruchsstoffträger an einem Brillengestell einer Sehbrille vorgesehen, welche von dem Organismus bzw. der betroffenen Person getragen wird, anbringbar oder darin integriert. Für einen Brillenträger kann demnach die erfindungsgemäße Vorrichtung zur Behandlung von Über- oder Untergewicht an einem Brillengestell seiner Sehbrille mechanisch angebracht werden, sodass die Behandlung in bequemer Weise durchgeführt wird.

In einer weiteren möglichen alternativen Ausführungsform der erfindungsgemäß Vorrichtung ist die tragbare Einheit der Vorrichtung mit der Aufnahmekammer für den Duftstoffträger an einem akustischen Hörgerät, welches von dem Organismus bzw. der Person getragen wird, anbringbar oder darin integriert.

Auch diese Ausführungsform bietet den Vorteil, dass die erfindungsgemäße Vorrichtung zur Behandlung der Gewichtsstörung in bequemer Weise kaum merklich für die betroffene Person bzw. den betroffenen Patienten getragen werden kann.

In einer weiteren möglichen Ausführungsform der erfindungsgemäßen Vorrichtung beträgt der räumliche Abstand zwischen der Membran der Aufnahmekammer für den Geruchsstoffträger und einem Geruchsorgan des betroffenen Organismus weniger als 20 cm. Die unmittelbare räumliche Nähe zwischen der Membran und dem Geruchsorgan bietet den Vorteil, dass der freigesetzte Geruchsstoff in hoher Konzentration zu dem Geruchsorgan des Organismus gelangen kann. Dieser räumliche Abstand ist zudem weitestgehend konstant, sodass die Konzentration in geeigneter Weise einstellbar ist.

In einer weiteren möglichen Ausführungsform der erfindungsgemäßen Vorrichtung ist der von dem Geruchsstoffträger freigesetzte Geruchsstoff geeignet, die Nahrungsaufnahme des Organismus zu reduzieren. Bei dieser Ausführungsform führt der freigesetzte Geruchsstoff langfristig zu einer Verminderung des Körpergewichtes eines übergewichtigen Organismus.

In einer möglichen Ausführungsform weist der freigesetzte Geruchsstoff einen Vanillegeruchsstoff auf, welcher geeignet ist, die Nahrungsaufnahme des Organismus zu reduzieren.

In einer weiteren möglichen alternativen Ausführungsform der erfindungsgemäßen Vorrichtung ist der von dem Geruchsstoffträger freigesetzte Geruchsstoff geeignet, die Nahrungsaufnahme des Organismus zu erhöhen bzw. zu stimulieren. Bei dieser Ausführungsform wird das Körpergewicht des Organismus aufgrund des Geruchsstoffes langfristig erhöht.

In einer weiteren möglichen Ausführungsform der erfindungsgemäßen Vorrichtung weist die Aufnahmekammer eine Injektionsöffnung zur Injektion eines Geruchsstoffes in den darin enthaltenen Geruchsstoffträger auf. Die Injektion kann beispielsweise mithilfe einer Spritze oder einer Kanüle erfolgen.

Die Erfindung schafft ferner einen Geruchsstoffträger zum Einbringen in eine Aufnahmekammer einer erfindungsgemäßen Vorrichtung zur Behandlung eines Organismus gegen Über- oder Untergewicht, wobei der Geruchsstoffträger aus einem saugfähigen Material besteht, das mit einem Geruchsstoff versehen ist.

In einer möglichen Ausführungsform des erfindungsgemäßen Geruchsstoffträgers ist dieser kugelförmig, kapselförmig oder tablettenförmig ausgebildet.

Dies bietet den besonderen Vorteil, dass der Geruchsstoffträger in einfacher Weise handhabbar ist.

In einer weiteren möglichen Ausführungsform des erfindungsgemäßen Geruchsstoffträgers ist dieser austauschbar und farbkodiert, wobei ein Farbton die Art und Konzentration des Geruchsstoffes innerhalb des Geruchsstoffträgers sowie dessen Auswirkung auf die Nahrungsaufnahme des Organismus angibt.

Diese Ausführungsform erlaubt eine gezieltere Verabreichung des Geruchstoffes in einer angemessenen Dosierung und Konzentration an das Geruchsorgan des Organismus. Darüber hinaus wird die Wahrscheinlichkeit von fehlerhaften oder irrtümlichen Behandlungen reduziert.

Im Weiteren werden mögliche Ausführungsformen der erfindungsgemäßen Behandlungsvorrichtung zur Behandlung eines Organismus gegen Über- oder Untergewicht sowie des erfindungsgemäßen Geruchsstoffträgers unter Bezugnahme auf die beigefügten Figuren näher erläutert.

Es zeigen:
- Figur 1:: eine mögliche Ausführungsform der erfindungsgemäßen Behandlungsvorrichtung;
- Figur 2:: ein weiteres Ausführungsbeispiel einer erfindungsgemäßen Behandlungsvorrichtung mit einem Klapp- und Drehmechanismus;
- Figur 3:: eine weitere mögliche Ausführungsform einer erfindungsgemäßen Behandlungsvorrichtung mit einem Klapp- und Drehmechanismus;
- Figur 4:: eine weitere mögliche Ausführungsform einer erfindungsgemäßen Behandlungsvorrichtung mit einer Injektionsöffnung;
- Figur 5:: ein weiteres Ausführungsbeispiel einer erfindungsgemäßen Behandlungsvorrichtung zum Anbringen an ein Brillengestell; und
- Figuren 6A, 6B:: mögliche Anwendungsbeispiele für die Anwendung einer erfindungsgemäßen Behandlungsvorrichtung zur Behandlung eines Organismus gegen Über- oder Untergewicht.

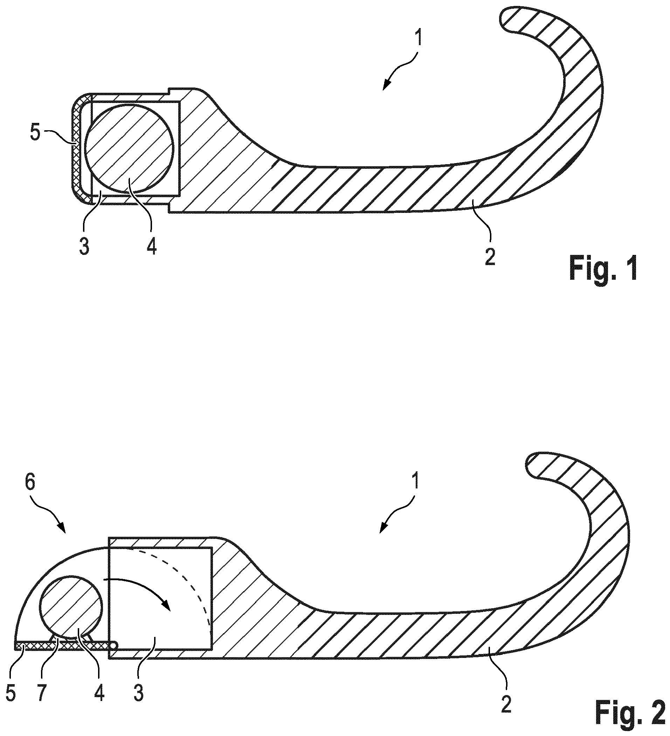

Figur 1 zeigt schematisch ein Ausführungsbeispiel einer erfindungsgemäßen Behandlungsvorrichtung 1 zur Behandlung eines Organismus gegen Über- oder Untergesicht. Die in Figur 1 dargestellte Vorrichtung 1 wird bei dem dargestellten Ausführungsbeispiel mithilfe eines Bügels 2 an einer Ohrmuschel einer Person angebracht. Dabei wird der U-förmige Bügel hinten an einer Ohrmuschel eines Ohres der Person befestigt bzw. angebracht. Der U-förmige Bügel hintergreift die Ohrmuschel. In einer bevorzugten Ausführungsform besteht der Bügel 2 der Vorrichtung 1 aus einem elastischen bzw. flexiblen Material, welches an die Form der Ohrmuschel angepasst ist. Bei dem in Figur 1 dargestellten Ausführungsbeispiel befindet sich an der dem Ohrläppchen des Ohres zugewandten Seite der Vorrichtung 1 eine Aufnahmekammer 3 zur Aufnahme eines Geruchsstoffträgers 4. In dem in Figur 1 dargestellten Ausführungsbeispiel bildet der Bügel 2 eine tragbare Einheit, die sich an ihrem unteren distalen Ende ausweitet, wobei dort eine Aufnahmekammer 3 zur Aufnahme eines Geruchsstoffträgers 4 vorgesehen ist. Bei dem in Figur 1 dargestellten Ausführungsbeispiel weist die Aufnahmekammer 3 eine Membran 5 auf, durch die ein von dem Geruchsstoffträger 4 freigesetzter Geruchsstoff an die Umgebungsluft abgegeben wird und von dort zu einem Geruchsorgan des Organismus bzw. der Person gelangt. Der freigesetzte Geruchsstoff beeinflusst eine Nahrungsaufnahme des Organismus. Bei dem Organismus handelt es sich beispielsweise um einen Menschen bzw. einen Patienten. Alternativ kann es sich bei dem Organismus auch um ein Tier, insbesondere um ein Haustier oder Nutztier handeln. Bei dem in Figur 1 dargestellten Ausführungsbeispiel verfügt die Vorrichtung 1 über eine Aufnahmekammer 3 zur Aufnahme mindestens eines Geruchsstoffträgers 4. In einer alternativen Ausführungsform kann die Vorrichtung mehrere Aufnahmekammern für verschiedene Geruchsstoffträger beinhalten. Der freigesetzte Geruchsstoff kann die Nahrungsaufnahme des Organismus beeinflussen, wobei der Geruchsstoff geeignet ist, entweder die Nahrungsaufnahme des Organismus zu erhöhen oder zu reduzieren. Der Geruchsstoffträger 4 besteht in einer möglichen Ausführungsform aus einem saugfähigen Material, das mit einem Geruchsstoff versehen ist. Beispielsweise kann der Geruchsstoffträger einen Trägerkörper aufweisen, der aus einem saugfähigen Material besteht, welches mit einem Duftstoff bzw. Geruchsstoff getränkt wird.

Figur 2 zeigt ein weiteres Ausführungsbeispiel einer erfindungsgemäßen Behandlungsvorrichtung 1 zur Behandlung eines Organismus gegen Über- oder Untergewicht. Bei dieser Ausführungsform weist die Aufnahmekammer 3 einen Klapp- oder Drehmechanismus zum Einbringen, Entfernen oder Austauschen eines mit einem Geruchsstoff versehenen Geruchsstoffträgers 4 auf. In dem in Figur 2 dargestellten Ausführungsbeispiel kann die Membran 5 von einem Drehmechanismus 6 um ihre Achse gedreht bzw. geklappt werden. Der Geruchsstoffträger 4 wird in eine Aufnahmekontur 7 eingesetzt und durch Verschwenken der Membran 5 in die Aufnahmekammer 3 der Vorrichtung 1 eingesetzt. Bei dem in Figur 2 dargestellten Ausführungsbeispiel ist der Geruchsstoffträger 4 kugelförmig ausgebildet. Bei weiteren möglichen Ausführungsformen ist der Geruchsstoffträger 4 beispielsweise auch kapselförmig oder tablettenförmig ausgebildet, um eine einfache Handhabe durch den Patienten zu gewährleisten.

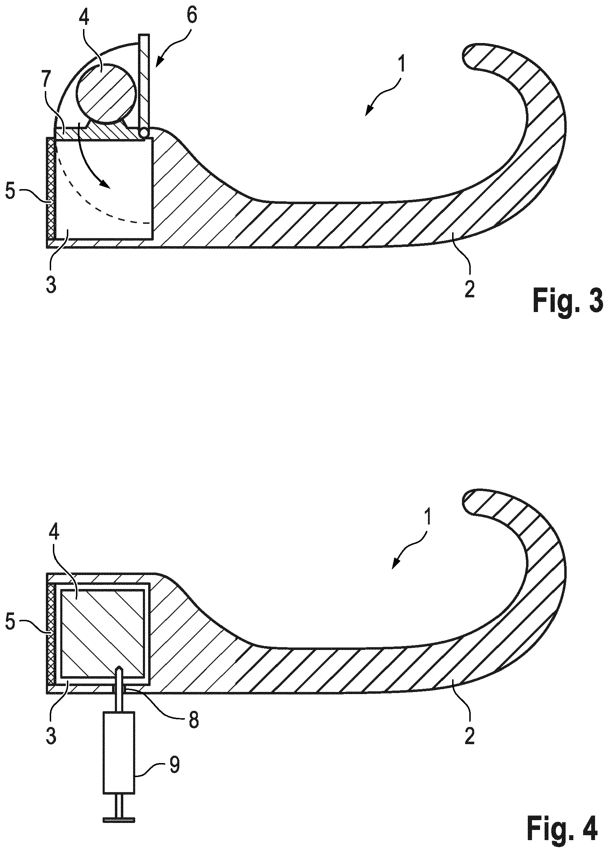

Figur 3 zeigt ein weiteres Ausführungsbeispiel einer erfindungsgemäßen Behandlungsvorrichtung 1. Bei dieser Ausführungsform ist der Klapp- und Drehmechanismus nicht stirnseitig im Bereich der Membran 5 angebracht, sondern seitlich. Beispielsweise kann ein Patient die Behandlungsvorrichtung 1 von seinem Ohr entfernen und einen Geruchsstoffträger 4 in eine Aufnahmekontur 7 mittels des Klapp- und Drehmechanismus 6 einsetzen und in die Aufnahmekammer 3 hineinschwenken. Der in die Aufnahmekammer 3 eingeführte Geruchsstoffträger 4 gibt dann über die Membran 5 den freigesetzten Geruchsstoff an die Umgebungsluft ab.

Figur 4 zeigt ein weiteres mögliches Ausführungsbeispiel einer erfindungsgemäßen Behandlungsvorrichtung 1 zur Behandlung eines Organismus gegen Über- oder Untergewicht. Bei dieser Ausführungsform verfügt die Aufnahmekammer 3 über eine Injektionsöffnung 8 zur Injektion eines Geruchsstoffes in den darin enthaltenen Geruchsstoffträger 4. Dies kann beispielsweise mithilfe einer Injektionsspritze 9 erfolgen. Der in der Aufnahmekammer 3 befindliche Geruchsstoffträger 4 kann entweder darin integriert sein oder alternativ mithilfe eines Klapp- und Drehmechanismus 6 ausgetauscht werden. Ein ausgetrockneter integrierter Geruchsstoffträger 4 kann mithilfe der Injektionsspritze 9 mit Geruchsstoff angereichert werden.

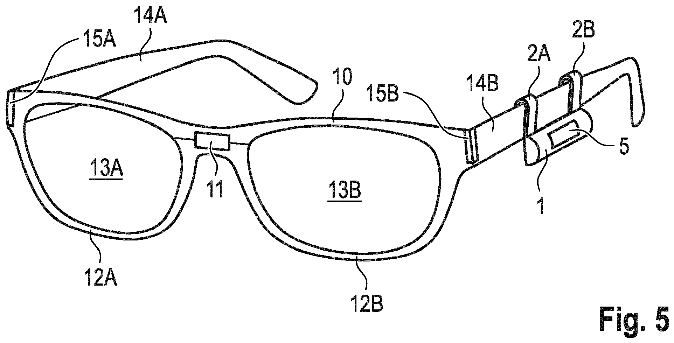

Figur 5 zeigt ein weiteres Ausführungsbeispiel einer erfindungsgemäßen Behandlungsvorrichtung 1 zur Behandlung eines Organismus gegen Über- oder Untergewicht. Bei dieser Ausführungsform ist die tragbare Einheit der Vorrichtung 1 mithilfe von elastischen Bügeln 2A, 2B an einem Seitenbügel 14B einer Sehbrille 10 angebracht. Die Sehbrille 10 hat einen Nasenbügel 11 mit je zwei Glasrahmen 12A, 12B für zwei Brillengläser 13A, 13B. Die beiden Seitenbügel 14A, 14B sind über Scharniere 15A, 15B mit den Brillenglasrahmen 12A, 12B verbunden. An den Seitenbügeln 14A, 14B der Sehbrille 10 können jeweils eine tragbare Einheit einer erfindungsgemäßen Behandlungsvorrichtung 1 beispielsweise mithilfe von elastischen Bügeln angebracht werden. Diese verfügen jeweils über mindestens eine Aufnahmekammer 3 zur Aufnahme eines Geruchsstoffträgers 4, der einen Geruchsstoff über eine Membran 5 an die Umgebungsluft freisetzt. Von dort gelangt der freigesetzte Geruchsstoff zur Nase bzw. zum Geruchsorgan der Person. In einer möglichen Ausführungsform kann die tragbare Einheit mit der Aufnahmekammer 3 entlang der Seitenbügel 14A, 14B lateral verschoben werden, um so die Konzentration des Geruchsstoffes im Bereich des Geruchsorgans einzustellen bzw. zu beeinflussen. Je geringer die räumliche Entfernung zwischen dem Geruchsorgan und der Membran 5 ist, desto höher ist die Konzentration des freigesetzten Geruchsstoffes im Bereich des Geruchsorganes bzw. der Nase des Patienten.

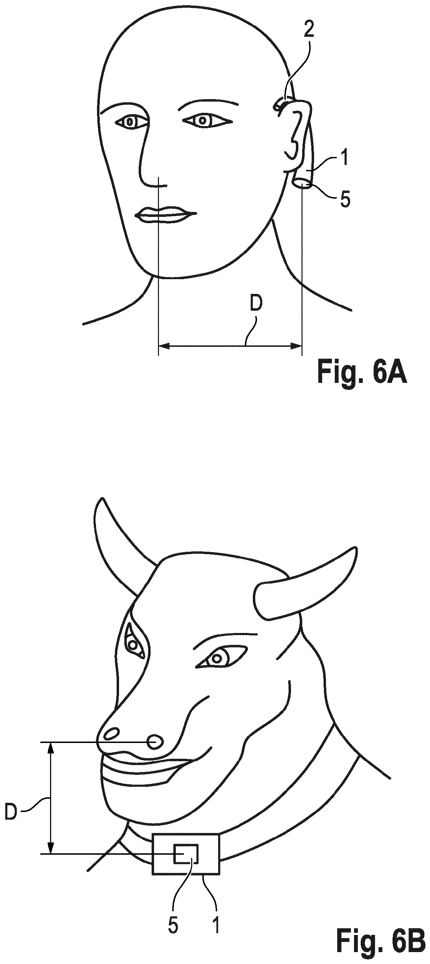

Figur 6A, 6B zeigen mögliche Anwendungsbeispiele für eine erfindungsgemäße Vorrichtung zur Behandlung eines Organismus gegen Über- oder Untergewicht.

Bei dem in Figur 6A schematisch dargestellten Anwendungsbeispiel handelt es sich bei dem Organismus um eine Person. Bei dem dargestellten Anwendungsbeispiel wird die tragbare Einheit an der Ohrmuschel des Ohres der Person mithilfe eines Bügels 2 angebracht. Der Abstand D zwischen der Membran 5 der tragbaren Einheit der Behandlungsvorrichtung 1 und dem Geruchsorgan bzw. der Nase der Person ist weitestgehend konstant. In einer möglichen Ausführungsform beträgt der Abstand D zwischen der Membran 5 der Aufnahmekammer und dem Geruchsorgan bzw. der Nase des Patienten weniger als 20 cm. Somit kann Geruchsstoff in ausreichender Konzentration zu dem Geruchsorgan der Person kontinuierlich gelangen.

Bei dem in Figur 6B schematisch dargestellten Ausführungsbeispiel handelt es sich bei dem Organismus um ein Nutztier, beispielsweise eine Kuh. In dieser Ausführungsform kann beispielsweise eine tragbare Einheit mit einer Aufnahmekammer für einen Geruchsstoffträger 4 an einem Halsband des Nutztieres befestigt sein. Der Abstand zwischen der Membran 5 und dem Geruchsorgan des Nutztieres ist ebenfalls konstant. In einer möglichen Ausführungsform ist der Abstand zwischen der Membran 5 und dem Geruchsorgan des Nutztieres geringer als 20 bis 30 cm. Die tragbare Einheit der Behandlungsvorrichtung 1 kann auch an anderer Stelle des Kopfes des Nutztieres angebracht werden, beispielsweise an dessen Hörnern. Das gleichzeitige Anbringen von mehreren Behandlungsvorrichtungen 1 an einem Kopf der zu behandelnden Person bzw. an einem Kopf des Nutztieres ist möglich. Beispielsweise werden gleichzeitig zwei Vorrichtungen an beiden Ohren der in Figur 6A dargestellten Person angebracht. Hierdurch kann die Konzentration des Geruchsstoffs, welcher zu dem Geruchsorgan der Person gelangt, erhöht werden.

Leidet die betroffene Person an Übergewicht, wird in die Aufnahmekammer 3 der Behandlungsvorrichtung 1 ein Geruchsstoffträger 4 eingesetzt, der einen Geruchsstoff freisetzt, der die Nahrungsaufnahme durch die Person reduziert. Bei diesem Geruchsstoff handelt es sich beispielsweise um einen Vanillegeruchsstoff. Zur Applikation von Vanilleduft konnte in Versuchen nachgewiesen werden, dass die betroffenen Testpersonen signifikant niedrige Mengen an Süßspeisen zu sich nehmen. Litt die betroffene Person umgekehrt unter Magersucht bzw. Untergewicht, kann durch geeignete Geruchsstoffe ein Hungergefühl stimuliert werden, sodass die betroffene Person zusätzlich Nahrung zu sich nimmt und somit an Körpergewicht zunimmt.

Haustiere, beispielsweise Katzen oder Hunde, weisen in vielen Fällen ebenfalls Übergewicht auf. In diesem Fall kann beispielsweise an einem Halsband des Haustieres eine tragbare Einheit mit einer Aufnahmekammer 3 zur Aufnahme eines Geruchsstoffträgers 4 vorgesehen werden. Dieser Geruchsstoff kann die Nahrungsaufnahme durch das betroffene Haustier reduzieren, sodass sein Körpergewicht vermindert wird.

In einer möglichen Ausführungsform werden die Geruchsstoffträger 4 aus einer Packung entnommen. Die Verpackung ist vorzugsweise luftdicht, sodass der in dem Geruchsstoffträger 4 enthaltene Geruchsstoff nicht entweichen kann, bevor er aus der Verpackung entnommen und in die Aufnahmekammer 3 der tragbaren Einheit eingesetzt worden ist. Beispielsweise befindet sich eine Vielzahl von Geruchsstoffträgern 4 in einer Vakuumverpackung. Nach Aufreißen der Verpackung der Packung kann die Person eine oder mehrere Geruchsstoffträger 4 entnehmen und in die Aufnahmekammer 3 einer Behandlungsvorrichtung 1 einsetzen. Alternativ können die Geruchsstoffträger 4 selbst mit einer luftdichten Packungsfolie umgeben werden, welche bei Bedarf abgezogen wird. Die Person nimmt den folienummantelten Geruchsstoffträger 3, entfernt die Schutzfolie, bevor sie den Geruchsstoffträger 4 in die Aufnahmekammer 3 der Behandlungsvorrichtung 1 einsetzt.

Der Geruchsstoffträger 4 weist vorzugsweise ein saugfähiges Trägermaterial auf. Bei dem Geruchsstoffträger handelt es sich beispielsweise um ein Tonkügelchen oder um eine Schaumstoffkugel. Der Geruchsstoffträger 4 ist vorzugsweise mit einem Geruchsstoff versehen bzw. damit getränkt.

Die tragbare Einheit, wie sie beispielsweise in Figuren 1 bis 4 dargestellt ist, besteht neben dem elastischen Bügel 2 in ihren erweiterten Bereich aus einem festeren Material, beispielsweise Polypropylen oder Polyvinyl. Die Membran 5 besteht in einer möglichen Ausführungsform aus perforiertem Kunststoff. Alternativ kann auch ein engmaschiges Metallgitter für das Freisetzen des Geruchsstoffes vorgesehen sein. In einer möglichen Ausführungsform wird die erfindungsgemäße Vorrichtung 1 in ein akustisches Hörgerät integriert oder daran angebracht. Alternativ kann die erfindungsgemäße Behandlungsvorrichtung 1 auch in das Brillengestell einer Sehbrille 10 integriert werden oder daran angebracht werden, wie in Figur 5 dargestellt.

In einer bevorzugten Ausführungsform sind die in die Aufnahmekammer 3 einsetzbaren Geruchsstoffträger 4 austauschbar und farbkodiert. Ein Farbton kann dabei die Art und Konzentration des Geruchsstoffes innerhalb des Geruchsstoffträgers 4 sowie dessen Auswirkung auf die Nahrungsaufnahme des betroffenen Organismus angeben. Beispielsweise gibt die Farbe des Geruchsstoffträgers an, welchen Geruchsstoff der Geruchsstoffträger freisetzt. Weiterhin kann der Farbton bzw. die Farbtiefe der Farbe angeben, welche Konzentration der abgegebene Geruchsstoff aufweist. Auf diese Weise können Verwechslungen bei der Handhabe der Geruchsstoffträger 4 vermieden werden. Darüber hinaus kann die Person die Geruchsstoffkonzentration für sich einstellen.

Nach einer gewissen Tragezeit kann die Person den in der Aufnahmekammer 3 befindlichen Geruchsstoffträger 4 gegen einen anderen Geruchsstoffträger austauschen.

Weitere Ausführungsformen sind möglich, bei einer möglichen Ausführungsform verfügt die tragbare Einheit über eine Schließeinheit zum Abdecken bzw. Verschließen der Geruchsstoffabgabemembran 5, um ein Austrocknen des Geruchsstoffträgers 4 innerhalb der Aufnahmekammer 3 zu verhindern, insbesondere wenn die Behandlungsvorrichtung 1 durch die Person getragen wird. Über eine Injektionsöffnung kann zudem der eingesetzte Geruchsstoffträger 4 mit zusätzlichem Geruchsstoff versehen werden. Der Geruchsstoff wird beispielsweise über eine Kanüle aus einem Behälter entnommen, in dem sich flüssiger Geruchsstoff befindet. In einer weiteren möglichen Ausführungsvariante verfügt die tragbare Einheit über eine integrierte Energiequelle, beispielsweise Batterie, die Strom liefert, um den Geruchsstoffträger 4 zur Freisetzung des Geruchsstoffes zu erwärmen. In einer weiteren möglichen Ausführungsform wird die tragbare Einheit durch eine hinten an der Ohrmuschel entlanglaufende Gummiröhre, eine sich an die Haut anpassendes flexibles Material, ähnlich wie bei einem Kopfhörer gebildet.

## Patentansprüche

1. Vorrichtung (1) zur Behandlung eines Organismus gegen Über- oder Untergewicht mit einer an einem Körperteil des Organismus tragbaren Einheit, die mindestens eine Aufnahmekammer (3) zur Aufnahme eines Geruchsstoffträgers (4) aufweist, der einen Geruchsstoff zur Beeinflussung einer Nahrungsaufnahme des Organismus freisetzt.

2. Vorrichtung nach Anspruch 1,
wobei die Aufnahmekammer (3) eine Membran aufweist, durch die der von dem Geruchsstoffträger (4) freigesetzte Geruchsstoff an die Umgebungsluft abgegeben wird und zu einem Geruchsorgan des Organismus gelangt.

3. Vorrichtung nach Anspruch 1 oder 2,
wobei die Aufnahmekammer (3) einen Klapp- oder Drehmechanismus (6) zum Einbringen, Entfernen oder Austauschen eines mit dem Geruchsstoff versetzten Geruchsstoffträgers (4) aufweist.

4. Vorrichtung nach einem der vorangehenden Ansprüche 1 bis 3,
wobei die tragbare Einheit mit der Aufnahmekammer (3) an einem Kopf des Organismus anbringbar ist.

5. Vorrichtung nach einem der vorangehenden Ansprüche 1 bis 4,
wobei die tragbare Einheit an einer Ohrmuschel oder an einem Ohrläppchen eines Ohres des Organismus anbringbar ist.

6. Vorrichtung nach einem der vorangehenden Ansprüche 1 bis 4,
wobei die tragbare Einheit mit der Aufnahmekammer (3) an einem Brillengestell einer Sehbrille (10), welche von dem Organismus getragen wird, anbringbar oder darin integriert ist.

7. Vorrichtung nach einem der vorangehenden Ansprüche 1 bis 4,
wobei die tragbare Einheit mit der Aufnahmekammer (3) an einem akustischen Hörgerät, welches von dem Organismus getragen wird, anbringbar oder darin integriert ist.

8. Vorrichtung nach einem der vorangehenden Ansprüche 2 bis 7,
wobei ein räumlicher Abstand zwischen der Membran (5) der Aufnahmekammer (3) und einem Geruchsorgan des Organismus weniger als 20 cm beträgt.

9. Vorrichtung nach einem der vorangehenden Ansprüche 1 bis 8,
wobei der von dem Geruchsstoffträger (4) freigesetzte Geruchsstoff geeignet ist, die Nahrungsaufnahme des Organismus zu erhöhen oder zu reduzieren.

10. Vorrichtung nach Anspruch 9,
wobei der von dem Geruchstoffträger (4) freigesetzte Geruchsstoff einen Vanillegeruchsstoff aufweist, welcher geeignet ist, die Nahrungsaufnahme des Organismus zu reduzieren.

11. Vorrichtung nach einem der vorangehenden Ansprüche,
wobei die Aufnahmekammer (3) eine Injektionsöffnung (8) zum Injizieren eines Geruchsstoffes in den darin enthaltenen Geruchsstoffträger (4) aufweist.

12. Vorrichtung nach einem der vorangehenden Ansprüche,
wobei der die Vorrichtung (1) tragende Organismus ein Mensch oder ein Tier, insbesondere ein Haus- oder Nutztier, ist.

13. Geruchsstoffträger (4) zum Einbringen in eine Aufnahmekammer (3) einer Vorrichtung (1) nach einem der vorangehenden Ansprüche 1 bis 12,
wobei der Geruchsstoffträger (4) aus einem saugfähigen Material besteht, das mit einem Geruchsstoff versehen ist.

14. Geruchsstoffträger nach Anspruch 13,
wobei der Geruchsstoffträger (4) kugelförmig, kapselförmig oder tablettenförmig ausgebildet ist.

15. Geruchsstoffträger nach Anspruch 13 oder 14,
wobei der Geruchsstoffträger (4) austauschbar und farbkodiert ist, wobei ein Farbton die Art und Konzentration des Geruchsstoffes innerhalb des Geruchsstoffträgers (4) sowie dessen Auswirkung auf die Nahrungsaufnahme des Organismus angibt.
